# EUROPEAN PATENT SPECIFICATION

(11) **EP 1 574 519 B1**
(45) Date of publication and mention of the grant of the patent: **27.01.2010**
(21) Application number: 03741487.7
(22) Date of filing: 18.07.2003
(51) Int. Cl.: C07K 14/47, C07K 16/18, C12N 15/12, A61P 1/16, A61P 35/00, G01N 33/53

(54) **Transcription control factor ZHX3**
Transkriptionskontrollfaktor ZHX3
Facteur de commande de la transcription ZHX3

(30) Priority: 18.12.2002 JP 2002366512
(43) Date of publication of application: 14.09.2005
(73) Proprietor: Japan Science and Technology Agency, Kawaguchi-shi, Saitama 332-0012 (JP)
(72) Inventor: MIYAMOTO, Kaoru, Sakai-gun, Fukui 910-0337 (JP); YAMADA, Kazuya, Sakai-gun, Fukui 910-0337 (JP)
(74) Representative: Wilson Gunn
(86) International application number: PCT/JP2003/009164
(87) International publication number: WO 2004/055051

(56) References cited:
- WO-A2-01/57190
- YAMADA KAZUYA ET AL: "Functional analysis and the molecular dissection of zinc-fingers and homeoboxes 1 (ZHX1)." BIOCHEMICAL AND BIOPHYSICAL RESEARCH COMMUNICATIONS. 20 SEP 2002, vol. 297, no. 2, 20 September 2002 (2002-09-20), pages 368-374, XP002366208 ISSN: 0006-291X
- NINOMIYA TOSHIAKI ET AL: "Regulation of the alpha-fetoprotein gene by the isoforms of ATBF1 transcription factor in human hepatoma." HEPATOLOGY (BALTIMORE, MD.) JAN 2002, vol. 35, no. 1, January 2002 (2002-01), pages 82-87, XP002366209 ISSN: 0270-9139
- BARTHELEMY I ET AL: "zhx-1: a novel mouse homeodomain protein containing two zinc-fingers and five homeodomains." BIOCHEMICAL AND BIOPHYSICAL RESEARCH COMMUNICATIONS. 25 JUL 1996, vol. 224, no. 3, 25 July 1996 (1996-07-25), pages 870-876, XP002366210 ISSN: 0006-291X
- YAMADA KAZUYA ET AL: "Analysis of zinc-fingers and homeoboxes (ZHX)-1-interacting proteins: molecular cloning and characterization of a member of the ZHX family, ZHX3." THE BIOCHEMICAL JOURNAL. 1 JUL 2003, vol. 373, no. Pt 1, 1 July 2003 (2003-07-01), pages 167-178, XP002366211 ISSN: 0264-6021
- YAMADA K ET AL: "Sp family members and nuclear factor-Y cooperatively stimulate transcription from the rat pyruvate kinase M gene distal promoter region via their direct interactions." THE JOURNAL OF BIOLOGICAL CHEMISTRY. 16 JUN 2000, vol. 275, no. 24, 16 June 2000 (2000-06-16), pages 18129-18137, XP002366340 ISSN: 0021-9258
- YAMADA K ET AL: "Identification of proteins that interact with NF-YA" FEBS LETTERS, ELSEVIER, AMSTERDAM, NL, vol. 460, no. 1, 22 October 1999 (1999-10-22), pages 41-45, XP004342716 ISSN: 0014-5793
- OSAWA H ET AL: "Identification and Characterization of Basal and Cyclic AMP Response Elements in the Promoter of the Rat Hexokinase II Gene" JOURNAL OF BIOLOGICAL CHEMISTRY, AMERICAN SOCIETY OF BIOLOCHEMICAL BIOLOGISTS, BIRMINGHAM,, US, vol. 271, no. 29, 19 July 1996 (1996-07-19), pages 17296-17303, XP002050907 ISSN: 0021-9258
- YAMADA K. ET AL.: 'Identification of proteins that interact with NF-YA' FEBS LETT. vol. 460, no. 1, 1999, pages 41 - 45, XP004240608
- YAMADA K. ET AL.: 'Human ZHX1: cloning, chromosomal location and interaction with transcription factor NF-Y' BIOCHEM. BIOPHYS. RES. COMMUN. vol. 261, no. 3, 1999, pages 614 - 621, XP002964727

## Description

### Field of the Invention

This invention relates to a protein acting as a transcriptional suppressor and, more specifically to transcriptional regulator ZHX3 for genes specific to hepatoma.

### Prior Art

Transcription of pyruvate kinase (PK) M gene and type II hexokinase (HK II) gene, which are genes involved in the glycolytic pathway, are induced in hepatoma cells, but silent in normal hepatocytes. The common transcription factor for both genes is nuclear factor-Y (NF-Y). However, there are no differences in the expression of NF-Y between normal hepatocytes and hepatoma cells. Therefore, some interacting partners of NF-Y may be responsible for the gene expression specific to hepatoma cells.

Running a search for proteins interacting with subunit A (NF-YA), an important subunit of NF-Y, the inventors cloned ZHX1, which consists of 873 amino acids and contains two zinc-finger motifs (Znf) and five homeodomains (HD) (Yamada, K., Osawa, H., and Granner, D. K. (1999) FEBS Lett. 460, 41-45). ZHX1 belongs to the Znf class of the homeobox protein superfamily and the amino acid sequence between 272 and 564 that contains the HD1-HD2 region of human ZHX1 is required for an interaction with the N-terminal glutamine-rich AD of NF-YA (Yamada, K., Osawa, H., and Granner, D. K. (1999) FEBS Lett. 460, 41-45). Northern blotting of ZHX1 revealed that the ZHX1 transcripts were expressed ubiquitously (Yamada, K., Printz, R. L., Osawa, H., and Granner, D. K. (1999) Biochem. Biophys. Res. Commun. 261, 614-621; Hirano, S., Yamada, K., Kawata, H., Shou, Z., Mizutani, T., Yazawa, T., Kajitani, T., Sekiguchi, T., Yoshino, M., Shigematsu, Y., Mayumi, M., and Miyamoto, K. (2002) Gene 290, 107-114). The human ZHX1 gene is located on chromosome 8q, between markers CHLC.GATA50B06 and CHLC.GATA7G07 (Yamada, K., Printz, R. L., Osawa, H., and Granner, D. K. (1999) Biochem. Biophys. Res. Commun. 261, 614-621). The inventors reported that ZHX1 functions as a transcriptional repressor and is localized in the nuclei (Yamada, K., Kawata, H., Matsuura, K., Shou, Z., Hirano, S., Mizutani, T., Yazawa, T., Yoshino, M., Sekiguchi, T., Kajitani, T., and Miyamoto. K. (2002) Biochem. Biophys. Res. Comm. 279, 368-374).

A nucleotide sequence (GenBank/XM 029734) containing the sequence encoding a novel transcriptional regulator (ZHX3) discovered by the inventors and a part of the sequence (DDBJ/AB007855) have been registered to the parenthetic database. The former has been registered as a putative protein by linking portions of the sequence scattered on the database and is suggestive of coding a certain protein. The latter is a partially cloned sequence and is without a functional analysis. Transcriptional repressor activity of the protein encoded by these disclosed nucleotide sequences has neither been known nor been guessed based on the disclosed information.

### Problems to be solved by the Invention

To determine the biological role of ZHX1, the inventors examined whether ZHX1 interacts with protein(s) other than NF-YA and regulates gene transcription. Using a yeast two-hybrid system, the inventors conducted a search for ZHX1-interacting proteins in rat liver and ovarian granulosa cell cDNA libraries.

### Means to solve the Problems

As the result of the search, nuclear proteins, such as ZHX1, transcription cofactors, DNA-binding proteins, zyxin, and androgen-induced aldolase reductase, 11-19 lysine-rich leukemia gene, as well as other unknown proteins, were cloned and a novel protein was found. Molecular cloning and determination of the nucleotide sequence of the full-length cDNA encoding the novel protein revealed that it consists of the 956 amino acid residues (SEQ ID NO: 1) and contains, like ZHX1, two zinc-finger (Znf) motif and five homeodomains (HDs).

The inventors concluded that the protein forms the ZHX family with ZHX1 and denoted it ZHX-3. It is deemed that the proteins of ZHX family may be involved in gene regulation by forming homodimers or heterodimers by interacting with each other.

ZHX3 not only dimerizes with both ZHX1 and ZHX3, but also interacts with the activation domain (AD) of NF-YA. Further analysis revealed that ZHX3 is a ubiquitous transcriptional repressor that is localized in nuclei and functions as a dimer.

Therefore, according to one aspect of the present invention there is provided a protein or a peptide having a transcriptional repressor activity for use as a medicament to treat hepatoma, comprising: (a) a protein or a peptide having an amino acid sequence of SEQ ID NO: 1; (b) a protein or peptide having an amino acid sequence with at least 85% similarity to the amino acid sequence of SEQ ID NO: 1; (c) a protein or peptide comprising, the functional domain consisting of amino acids 303-502 of the amino acid sequence of SEQ ID NO: 1; (d) a protein or peptide comprising an amino acid sequence with at least 85% similarity to the functional domain consisting of amino acids 303-502 of SEQ ID NO: 1.

The similarities in the amino acid sequences of ZHX3 between human and mouse is 85.3% and that between human and rat is 87.3%. The amino acid sequence of rat ZHX3 is shown in SEQ ID NO: 2. The rat-type amino acid sequence corresponds to amino acids 114-642 of human ZHX3 amino acid sequence. Therefore, the proteins comprising amino acid sequence with more than 85% similarity to ZHX3 amino acid sequence (SEQ ID NO: 1) could be ascribed to the protein with the same transcriptional repressor activity to human ZHX3.

As shown by the examples to be hereinafter described, the sequence of amino acids 303-502 of the amino acid sequence of SEQ ID NO: 1 is the domain involved in the function of transcriptional repressor.

Preferably, the protein or peptide is a repressor of the transcription of type II hexokinase gene or pyruvate kinase M gene.
Since hepatoma cells depend on enhanced glycolysis, ZHX3 may repress, in normal hepatocytes, the expression of isoenzyme gene family specific to hepatoma cells, and the expression of ZHX3 may be lowered during malignant transformation or ZHX3 protein may be inactivated by modification. Therefore, once agents to detect ZHX3 or drugs to regulate the function are developed, these can be applied to diagnosis and treatment of hepatoma.

According to another aspect of the invention there is provided the use of a screening agent to screen drug agents with transcriptional repressor activity for treating hepatoma, wherein the screening agent comprises an antibody specific to a protein or a peptide of any of (a) to (d) as an effective component: (a) a protein or a peptide having an amino acid sequence of SEQ ID NO: 1; (b) a protein or a peptide having an amino acid sequence with at least 85% similarity to the amino acid sequence of SEQ ID NO: 1, and having a transcriptional repressor activity; (c) a protein or a peptide comprising the functional domain consisting of amino acids 303-502 of the amino acid sequence of SEQ ID NO: 1; (d) a protein or a peptide comprising an amino acid sequence with at least 85% similarity to the functional domain consisting of amino acids 303-502 of SEQ ID NO: 1 and having a transcriptional repressor activity.

### Brief Description of the Drawings

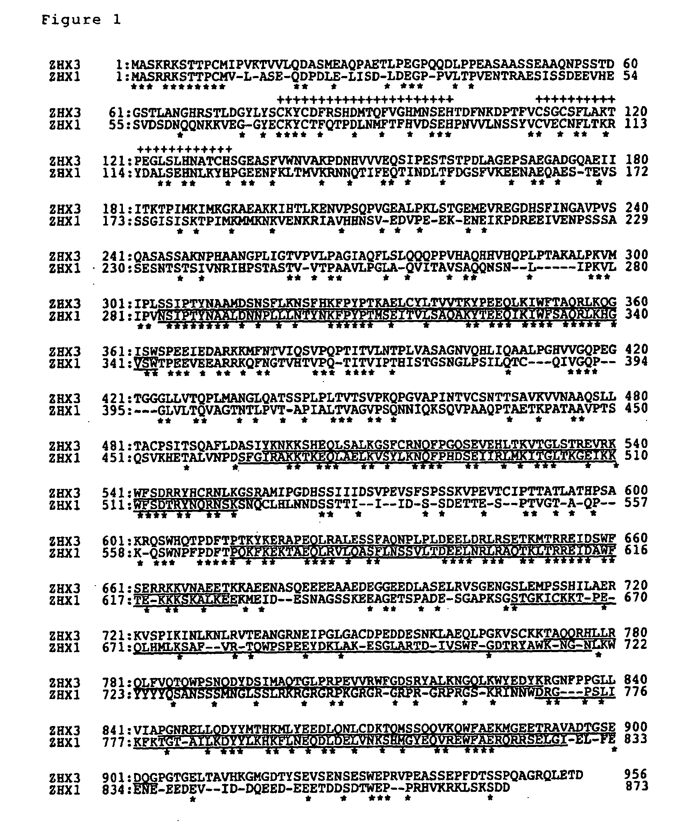

Figure 1 shows a comparison of deduced amino acid sequences of human ZHX3 with human ZHX1. The amino acid sequences of ZHX3 and ZHX1 are compared. Two Znf and five HD motifs were shown by plus signs and underlines, respectively. Asterisks indicate the similarities of the amino acid sequences. Dashed lines show gaps when corresponding amino acids are absent in each protein. The deduced amino acid sequences of clones G58, G23 and KIAA0395 correspond to the amino acid sequences between 114 and 642, between 242 and 615 and between 495 and 956 of human ZHX3, respectively, are included in Figure 1.

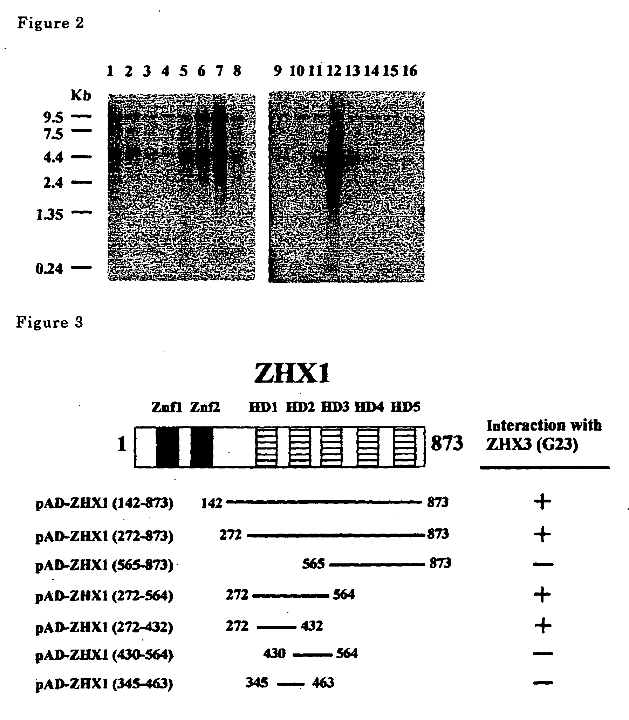

Figure 2 shows the tissue distribution of human ZHX3 mRNA. Each lane contains 2 µg of poly(A)⁺⁻RNA isolated from indicated tissues. Size markers are shown on the left in kb. Lane 1, heart; lane 2, brain; lane 3, placenta; lane 4, lung; lane 5, liver; lane 6, skeletal muscle; lane 7, kidney; lane 8, pancreas; lane 9, spleen; lane 10, thymus; lane 11, prostate; lane 12, testis; lane 13, ovary; lane 14, small intestine; lane 15, colon; and lane 16, leucocyte.
Figure 3 shows the identification of the minimal heterodimerization domain between ZHX1 and ZHX3 using the yeast two-hybrid system, GST pull-down assays. A schematic representation of human ZHX1 and the GAL4 AD-ZHX1 fusion constructs is depicted on the left. Znf and HD indicate a zinc-finger motif and a homeodomains, respectively. The + and - symbols indicate increased and unchanged levels of β - galactosidase activity, respectively, compared with a yeast harbouring a combination of pDBD-G23, expressing amino acids 242-488 of human ZHX3 fused to the GAL4 DBD, and pACT2.

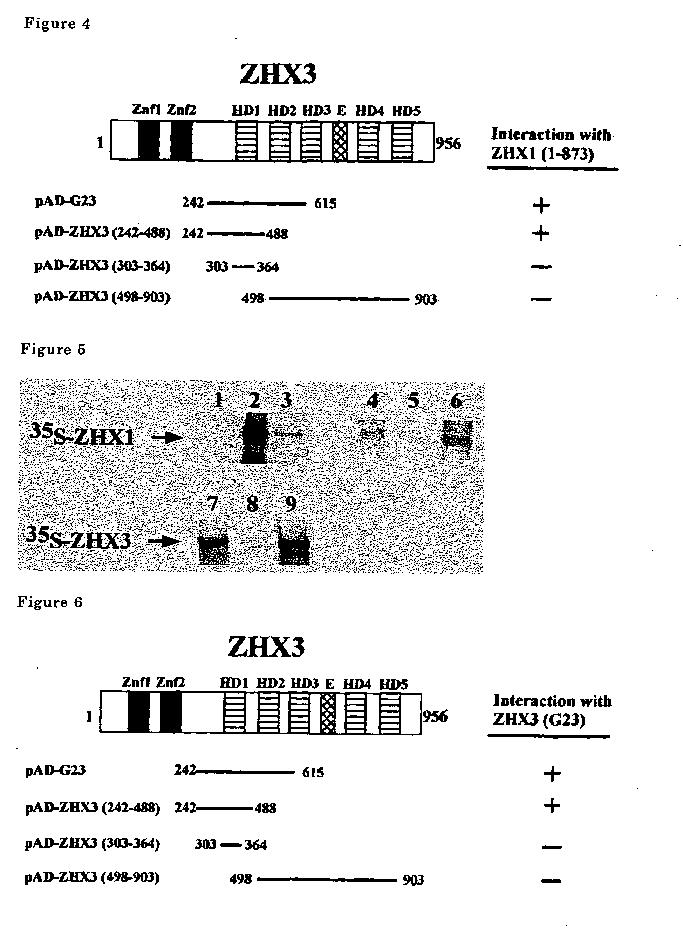

Figure 4 shows the identification of the minimal heterodimerization domain between ZHX1 and ZHX3 using the yeast two-hybrid system, GST pull-down assays. A schematic representation of human ZHX3 and the GAL4 AD-ZHX3 fusion constructs are depicted on the left. Znf, HD and E indicate a zinc-finger motif, a homeodomains, and a glutamic-acid-rich region, respectively. The + and - indicate increased and unchanged levels of β -galactosidase activity, respectively, compared with a yeast harbouring a combination of pDBD-ZHX1 (1-873), expressing the entire coding region of human ZHX1 fused to the GAL4 DBD, and pACT2.
Figure 5 shows the identification of the minimal heterodimerization domain between ZHX1 and ZHX3 using the yeast two-hybrid system, GST pull-down assays. In vitro-translated, ³⁵S-labelled full-length human ZHX1 or ZHX3 was incubated with Sepharose beads containing bound GST alone (lanes 1, 5 and 8) or amino acids 242-615 of ZHX3 (lane 3), or the entire coding sequences of human ZHX3 (lane 6) or ZHX1 (lane 9) protein fused to GST. The beads were washed thoroughly and the bound protein was eluted and analysed by SDS/PAGE (10% gel). Interaction signals were detected by an autoradiography. Lanes 2, 4 and 7, 10% of the protein added to the reactions shown in the other lanes were loaded.
Figure 6 shows the identification of the minimal homodimerization domain of ZHX3 using the yeast two-hybrid system, GST pull-down assays. A schematic representation of human ZHX3 and the GAL4 AD-ZHX3 fusion constructs are depicted on the left. Znf, HD and E indicate a zinc-finger motif, a homeodomains, and a glutamic-acid-rich region, respectively. The + and - symbols indicate the same as described for Figure 3.

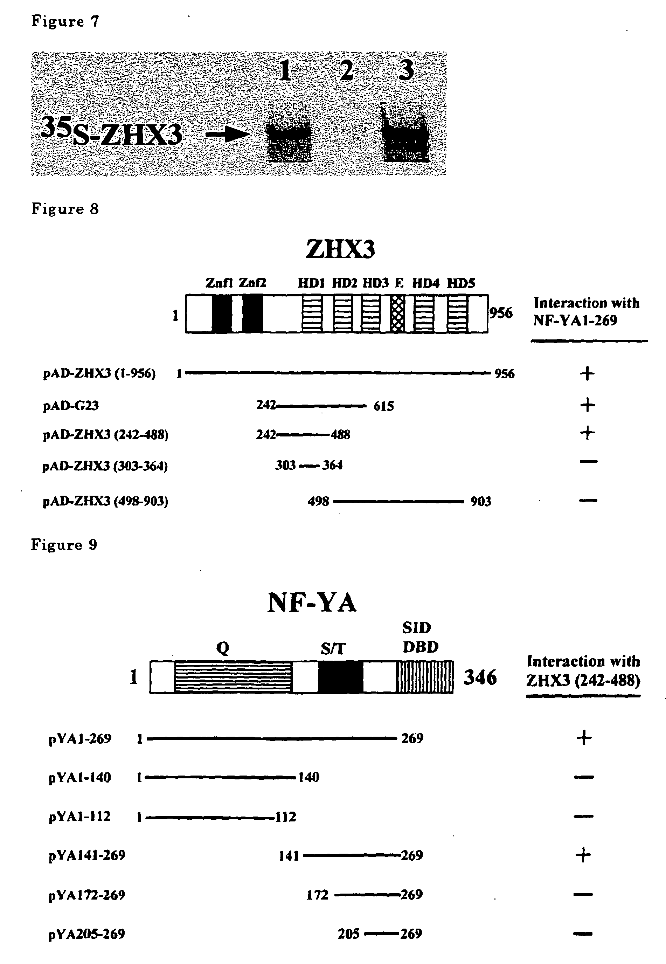

Figure 7 shows the identification of the minimal homodimerization domain of ZHX3 using the yeast two-hybrid system, GST pull-down assays. In vitro-translated, ³⁵S-labelled full-length human ZHX3 was incubated with Sepharose beads containing bound GST alone (lanes 2) or the entire coding sequences of human ZHX3 (lane 3) protein fused to GST. Procedures followed were the same to those described for Figure 5. Lane 1, 10% of the protein added to the reactions shown in the other lanes were loaded.
Figure 8 shows the interaction domain mapping between NF-YA and ZHX3 using the yeast two-hybrid system. A schematic representation of human ZHX3 and the GAL4 AD-ZHX3 fusion constructs are depicted on the left. Znf, HD and E indicate a zinc-finger motif, a homeodomains, and a glutamic-acid-rich region, respectively. The + and - indicate increased and unchanged levels of beta -galactosidase activity, respectively, compared with a yeast harbouring a combination of pYA1-269, expressing the NF-YA AD fused to the GAL4 DBD, and pACT2.
Figure 9 shows the interaction domain mapping between NF-YA and ZHX3 using the yeast two-hybrid system. Schematic diagram of NF-YA and its deletion mutants fused to the GAL4 DBD are depicted on the left. Q and S/T indicate glutamine- rich and serine/threonine-rich regions, respectively. SID and DBD indicate the subunit-interaction domain and the DNA binding domain, respectively. The + and - symbols indicate increased and unchanged levels of beta -galactosidase activity, respectively, compared with a yeast harbouring a combination of pDBD and pAD-ZHX3 (242-488), expressing amino acids 242-488 of human ZHX3 fused to the GAL4 AD.

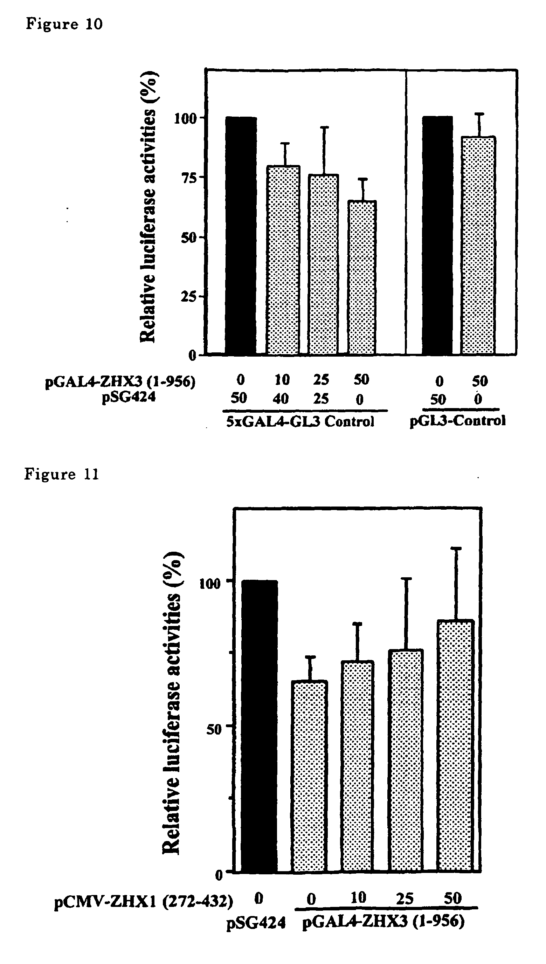

Figure 10 shows that ZHX3 is a transcriptional repressor. HEK293 cells were co-transfected with 2 ng of the pRL-CMV, 50 ng of the simian virus 40 (SV40) promoter-directed expression vector and 100 ng of the 5xGAL4-pGL3 Control or pGL3-Control reporter plasmid. pSG424 and pGAL4-ZHX3 (1-956) express GAL4 DBD alone and the entire coding sequence of human ZHX3 fused to the GAL4 DBD, respectively. A value of 100% was assigned to the promoter activity for the reporter plasmid in the presence of 50 ng of pSG424.
Figure 11 shows that ZHX3 is a transcriptional repressor. HEK293 cells were co-transfected with 2 ng of the pRL-CMV, 50 ng of the SV40 promoter-directed expression vector, 100 ng of the 5xGAL4-pGL3 Control reporter plasmid and the indicated amount of pCW-ZHX1 (242-432) expression plasmid. The total amount of plasmid (202 ng) was adjusted by the addition of the pcDNA3.1His-C2, if necessary. A value of 100% was assigned to the promoter activity of the reporter plasmid in the presence of 50 ng of pSG424 and 50 ng of pcDNA3.1His-C2. 48 h after transfection, cells were harvested and both firefly and sea pansy luciferase activities were determined. Firefly luciferase activities were normalized by the sea pansy luciferase activities in all experiments. Each column and bar represents the mean +/- S.D. from at least five transfection experiments.

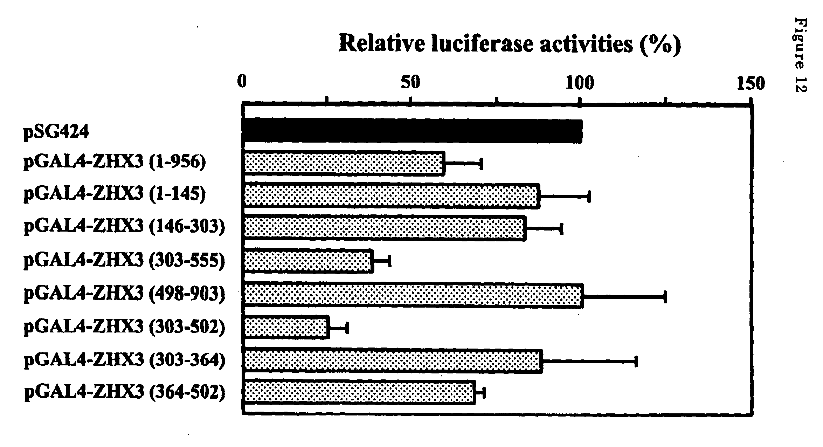

Figure 12 shows the minimal repressor domain of ZHX3. The pSG424 and various pGAL4-ZHX3 constructs express GAL4 DBD alone and various deletion mutants of human ZHX3 fused to the GAL4 DBD. Conditions were the same as described for Figure 10. Each column and bar represents the mean +/- S.D. from at least five transfection experiments.

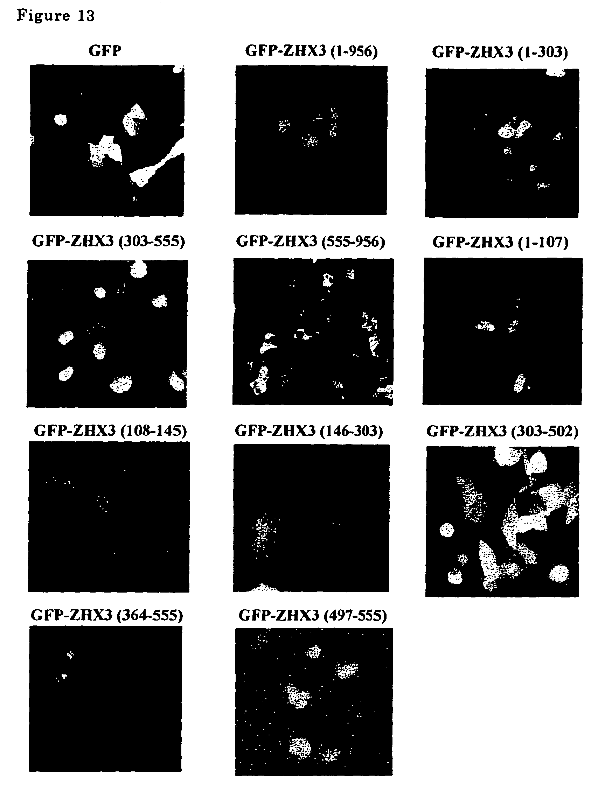

Figure 13 shows subcellular localization of ZHX3 in HEK293 cells and determination of NLSs. Expression plasmids (300 ng) encoding GFP alone or various truncated ZHX3 proteins fused to the C-terminus of GFP were transfected into HEK293 cells. At 48 h after transfection, the subcellular localization of GFP fusion proteins was observed. Constructs are given at the top of each panel.

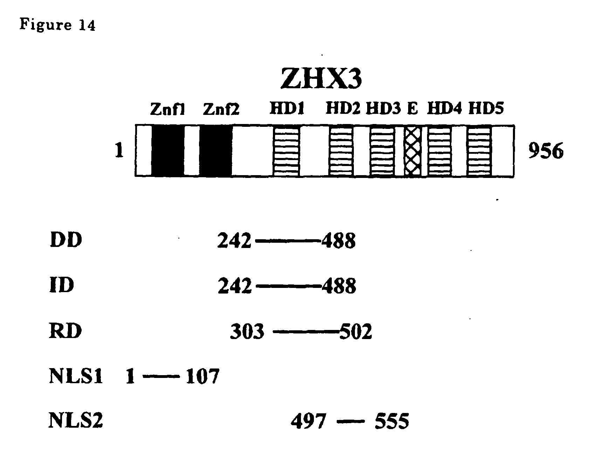

Figure 14 shows a schematic diagram of the functional domains of human ZHX3: Znf, zinc-finger motif; HD, homeodomain; E, glutamic acid-rich region; DD, dimerization domain; ID, interaction domain with NF-YA; RD, repressor domain; and NLS, nuclear-localization signal.

The following examples illustrate the invention. However, the examples are not intended to limit the scope of the invention.

### Examples

In the following examples, the yeast two-hybrid system, pDsRed1-C1, X- alpha - gal, human testis marathon-ready cDNA, Advantage 2 PCR kit, human Multiple Tissue Northern Blot and Blot II, ExpressHyb hybridization solution and pEGFP-C1 were all purchased from Clontech (Palo Alto, CA, U.S.A.). The pGEX-4T-2, pGEX-5X-1, α-²PdCTP (111 TBq/mmol), glutathione-Sepharose 4B and [³⁵S]methionine (37 TBq/mmol) were purchased from Amersham Pharmacia Biotech (Cleveland, OH, U.S.A.). HEK293 cells, a human embryonic kidney cell line, were purchased from the American Type Culture Collection (Manassas, VA, U.S.A.). Trizol reagent, Superscript II, pcDNA3.1His-C plasmid and LIPOFECTAMINE PLUS were purchased from Invitrogen (Groningen, Netherlands). ExTaq DNA polymerase, pT7Blue-T 2 vector and BcaBest DNA-labelling kit were obtained from TaKaRa Biomedicals (Kyoto, Japan). pGEM-T Easy vector, T7 TNT Quick-coupled transcription/translation system, pGL3-Control, pRL-CMV and dual-luciferase assay system were purchased from Promega (Madison, WI, U.S.A.). The Big Dye terminator FS cycle sequencing kit was purchased from Applied Biosystems Japan (Tokyo, Japan). TOPP3 cells were obtained from Stratagene (La Jolla, CA, U.S.A.). Qiagen plasmid kit were purchased from Qiagen (Hilden, Germany).

The following examples set out the experimental procedures used for the present invention.

### Reference example 1, Plasmid construction

The pACT2B1 plasmid has been prepared as described previously (Yamada, K., Osawa, H., and Granner, D. K. (1999) FEBS Lett. 460, 41-45). pAD-G23, a cloned G23 plasmid, was digested with EcoRI/XhoI or SfiI/BgIII and each fragment was subcloned into the EcoRI/XhoI sites of the pGEX-4T-2 vector or SfiI/BamHI sites of the pGBKT7 vector to obtain pGST-G23 and pDBD-G23, respectively.

A 250-bp EcoRI/HindIII fragment of pAD-G23 was subcloned into the EcoRI/HindIII sites of the pDsRed1-C1 to produce pDsRed-revereseG23 (HD1). The S-DsRed1C1-HindIII (SEQ ID NO: 3) and the As-DsRed1C1-SalI oligonucleotides (SEQ ID NO: 4) were then annealed, phosphorylated and inserted into the HindIII/SalI sites of the pDsRed1-C1 to obtain pDsRed1-C1E1. An EcoRI/BgIII fragment of the pDsRed-revereseG23 (HD1) was subcloned into the EcoRI/BamHI sites of the pDsRed1-C1E1 to produce pDsRed-rZHX3 (HD1). The EcoRI/XhoI fragment of the resultant plasmid was subcloned into the EcoRI/XhoI sites of the pACT2 to produce pAD-ZHX3 (242-488).

pBSII-KIAA0395 was a gift from Dr Takahiro Nagase (Kazusa DNA Research Institute, Chiba, Japan). A 1.2-kb SalI/ApaI fragment of the plasmid was subcloned into the SalI/ApaI sites of the pDsRed1-C1E1 to produce pDsRed-ZHX3 (HD2-5). The BglII/BamHI fragment of the resultant construct was subcloned into the BamHI site of pACT2B1 to obtain pAD-ZHX3 (498-903).

The pAD-ZHX1 (142-873), pAD-ZHX1 (272-873), pAD-ZHX1 (565-873), pAD-ZHX1 (272-564), pAD-ZHX1 (272-432), pAD-ZHX1 (430-564), pAD-ZHX1 (345-463), pDBD, pYA1-269, pYA1-140, pYA1-112, pYA141-269, pYA172-269 and pYA205-269 were constructed as described previously (Yamada, K., Osawa, H., and Granner, D. K. (1999) FEBS Lett. 460, 41-45; Yamada, K., Printz, R. L., Osawa, H., and Granner, D. K. (1999) Biochem. Biophys. Res. Commun. 261, 614-621).

Total RNA from HEK293 cells was prepared using the TRIZOL reagent according to the manufacturer's protocol. Reverse transcriptase PCRs (RT-PCRs) were performed as described previously with minor modification (Yamada, K., Printz, R. L., Osawa, H., and Granner, D. K. (1999) Biochem. Biophys. Res. Commun. 261, 614-621). PCR conditions were described previously except for the use of the ExTaq DNA polymerase(Yamada, K., Printz, R. L., Osawa, H., and Granner, D. K. (1999) Biochem. Biophys. Res. Commun. 261, 614-621). Combinations of S-hZHX3-ApaI2 (SEQ ID NO: 5) and As-hZHX3-STOP4 (SEQ ID NO: 6), S-hZHX3-NcoI (SEQ ID NO: 7) and AshZHX3-BsmBI2 (SEQ ID NO: 8), S-hZHX3-Met (SEQ ID NO: 9) and As-hZHX3-NcoI (SEQ ID NO: 10), S-hZHX3-HD1 (SEQ ID NO: 11) andAs-hZHX3-HD2 (SEQ ID NO: 12), S-hZHX3-HD1 (SEQ ID NO: 11) and As-hZHX3-1506 (SEQ ID NO: 13), S-hZHX3-1090 (SEQ ID NO: 14) and As-hZHX3-HD2 (SEQ ID NO: 12), S-hZHX3-HD2 (SEQ ID NO: 15) and As-hZHX3-HD2 (SEQ ID NO: 12), S-hZHX3-HD1 (SEQ ID NO: 11) and As-hZHX3-HD1 (SEQ ID NO: 16), and S-hZHX3N (SEQ ID NO: 17) and AshZHX3N (SEQ ID NO: 18), were used as primers.

These products were subcloned into the pGEM-T Easy vector to give pGEM-T Easy ZHX3 (ApaI/STOP), pGEM-T Easy ZHX3 (NcoI/BsmBI), pGEM-T Easy ZHX3 (Met/NcoI), pGEM-T Easy ZHX3 (HD1-2), pGEM-T Easy ZHX3 (HD1-1506), pGEM-T Easy ZHX3 (1090-HD2), pGEM-T Easy ZHX3 (HD2), pGEM-T Easy ZHX3 (HD1) and pGEM-T Easy ZHX3N, respectively.

GBKT7MCS1 (SEQ ID NO: 19) and GBKT7MCS2 oligonucleotides (SEQ ID NO: 20) were annealed, phosphorylated and subcloned into the EcoRI/BamHI sites of pGBKT7 to give pGBKT7B1. The ApaI/BamHI fragment of the pGEM-T Easy ZHX3 (ApaI/STOP) was subcloned into the ApaI/BamHI sites of the pDsRed-ZHX3 (HD2-5) to give pDsRed-ZHX3 (HD2-STOP). The EcoRI/BsmBI fragment of pGEM-T Easy ZHX3 (NcoI/BsmBI) was subcloned into the EcoRI/BsmBI sites of pDsRed-ZHX3 (HD2-STOP) to give pDsRed-ZHX3 (NcoI-STOP).

S-GBKT7-NdeI (SEQ ID NO: 21) and As-GBKT7-NcoI oligonucleotides (SEQ ID NO: 22) were annealed, phosphorylated and inserted into the NdeI/NcoI sites of pGBKT7B1 to give pGBKT7NEN. A 2-kb NcoI/BamHI fragment of the pDsRed-ZHX3 (NcoI-STOP) was subcloned into the NcoI/BamHI sites of the pGBKT7NEN to produce pGBKT7-ZHX3 (NcoI-STOP). pGEM-T Easy ZHX3 (Met/NcoI) was digested with NcoI and the 960-bp fragment was subcloned into the NcoI site of pGBKT7-ZHX3 (NcoI-STOP) to give pDBD-ZHX3 (1-956). The resultant plasmid was digested with BamHI, blunt-end ligated by the Klenow reaction, and then digested with EcoRI. The 2.9-kb fragment was subcloned into the EcoRI/SmaI sites of the pGEX-5X-1 vector to obtain pGST-ZHX3 (1-956). The EcoRI/XhoI fragment of the pGST-ZHX3 (1-956) was subcloned into the EcoRI/XhoI sites of the pACT2B1 to produce pAD-ZHX3 (1-956). PCRs were carried out using the pDBD-ZHX3 (1-956) as a template with the combination of S-hZHX3HD1 (SEQ ID NO: 11) and As-hZHX3-HD1-Eco (SEQ ID NO: 23) as primers. After digestion with EcoRI, the fragment was subcloned into the EcoRI site of pACT2B 1 to give pAD-ZHX3 (303-364).

The pSG424, pSG424B1, 5xGAL4-pGL3 Control and pEGFP-C1E1 plasmids have been described previously. The 2.9-kb EcoRI/BamHI fragment of the pDBD-ZHX3 (1-956) was subcloned into the EcoRI/BamHI sites of the pSG424B1 or pEGFP-C1E1 vector to give pGAL4-ZHX3 (1-956) and pGFP-ZHX3 (1-956), respectively. The BgIII/BamHI fragment of the pDsRed-ZHX3 (HD2-5) was subcloned into the BamHI site of pSG424B1 to give pGAL4-ZHX3 (498-903). The EcoRI/BamHI fragments of the pGEM-T Easy ZHX3 (HD1-2), pGEM-T Easy ZHX3 (HD1-1506), pGEM-T Easy ZHX3 (1090-HD2) and pGEM-T Easy ZHX3 (HD2) were subcloned into the EcoRI/BamHI sites of the pSG424B1 or pEGFP-C1E1 vector to produce pGAL4-ZHX3 (303-555), pGAL4-ZHX3 (303-502), pGFP-ZHX3 (303-555), pGFP-ZHX3 (303-502), pGFP-ZHX3 (364-555) and pGFP-ZHX3 (497-555), respectively. The EcoRI/BamHI fragment of the pGEM-T Easy ZHX3 (HD1) was subcloned into the EcoRI/BamHI sites of the pSG424B 1 vector to obtain pGAL4-ZHX3 (303-364).

PCRs were also carried out using pDBD-ZHX3 (1-956) as a template with the combination of S-hZHX3-Met3 (SEQ ID NO: 24) and As-hZHX3-909 (SEQ ID NO: 25), S-hZHX3-Met3 (SEQ ID NO: 24) and As-hZHX3-435 (SEQ ID NO: 26), S-hZHX3-436 (SEQ ID NO: 27) and As-hZHX3-909 (SEQ ID NO: 25), S-hZHX3-Met3 (SEQ ID NO: 24) and As-hZHX3-321 (SEQ ID NO: 28), S-hZHX3-322 (SEQ ID NO: 29) and As-hZHX3-435 (SEQ ID NO: 26), and S-hZHX3-1663 (SEQ ID NO: 30) and As-hZHX3-BsmBI-2 (SEQ ID NO: 31), as primers, and using the pGFP-ZHX3 (303-555) as a template with the combination of S-hZHX3-1090 (SEQ ID NO: 14) and AshZHX3-1506 (SEQ ID NO: 13) as primers. Amplified DNAs were also subcloned into the pGEM-T Easy or pT7Blue-2 T vector to produce pGEM-T Easy ZHX3 (Met/909), pGEM-T Easy ZHX3 (Met/435), pGEM-T Easy ZHX3 (436/909), pT7Blue-2 T ZHX3 (Met/321), pT7Blue-2 T ZHX3 (322/435), pGEM-T Easy ZHX3 (1663/2022) and pGEM-T Easy ZHX3 (1090/1506), respectively. The EcoRI/BamHI fragments of these plasmids were subcloned into the EcoRI/BamHI sites of the pSG424B or pEGFP-C1E1 vector to produce pGAL4-ZHX3 (1-145), pGAL4-ZHX3 (146-303), pGFP-ZHX3 (1-303), pGFP-ZHX3 (1-107), pGFP-ZHX3 (108-145) and pGFP-ZHX3 (146-303), respectively. The EcoRI/BamHI fragment of the pGEM-T Easy ZHX3 (1090/1506) was subcloned into the EcoRI/BamHI sites of the pSG424B1 to produce pGAL4-ZHX3 (364-502). The EcoRI/BsmBI fragment of the pGEM-T Easy ZHX3 (1663/2022) was subcloned into the EcoRI/BsmBI sites of the pDsRed-ZHX3 (HD2-STOP) to produce pDsRed-ZHX3 (1663-STOP). The EcoRI/BamHI fragment of the resultant plasmid was subcloned into the EcoRI/BamHI sites of the pEGFP-C1E1 to produce pGFP-ZHX3 (555-956).

HisCMCS1 (SEQ ID NO: 32) and HisCMCS2 oligonucleotides (SEQ ID NO: 33) were annealed, phosphorylated and subcloned into the KpnI/EcoRI sites of the pcDNA3.1His-C to give the peDNA3.1His-C2. The pGST-ZHX1 (272-432) was described previously. A 480-bp BamHI fragment of the pGST-ZHX1 (272-432) was subcloned into the BamHI site of the pcDNA3.1His-C2 to produce pCMV-ZHX1 (272-432).

The nucleotide sequences of all plasmids were confirmed using a DNA sequencer 3100 (Applied Biosystems).

### Reference example 2: Library screening

pDBD-ZHX1 (1-873) (pGBKT7-ZHX1 (1-873)), which expresses the entire coding sequence of human ZHX1 fused to the DNA-binding domain (DBD) of yeast transcription factor GAL4, and the construction of rat granulosa cell and liver cDNA libraries were described previously (Hirano, S., Yamada, K., Kawata, H., Shou, Z., Mizutani, T., Yazawa, T., Kajitani, T., Sekiguchi, T., Yoshino, M., Shigematsu, Y., Mayumi, M., and Miyamoto, K. (2002) Gene 290, 107-114 and others). AH109 yeast cells were transformed with the pDBD-ZHX1 (1-873) plasmid. The strain was used as a bait to screen cDNA libraries. A Tris/EDTA/lithium acetate-based high-efficiency transformation method was used for library screening (Yamada, K., Wang, J.-C., Osawa, H., Scott, D. K., and Granner, D. K. (1998) BioTechniques 24, 596-600). We plated approx. 1.5x10⁷ and 1.1x10⁷ independent clones of the liver and granulosa cell cDNA libraries on to histidine-, tryptophan-, leucine- and adenine-free synthetic dextrose plates supplemented with 4 mM 3-aminotriazole and X- α-gal, respectively. Thus 33 and 109 positive clones were obtained from the primary transformants, respectively. The yeast strain SFY526, which contains a quantifiable lacZ reporter, and either the pGBKT7 or pDBD-ZHX1 (1-873) plasmids, was transformed with plasmids isolated from positive clones in primary screening of the parent vector, pACT2. In the second screening, 16 and 25 clones from liver and granulosa cell cDNA libraries, respectively, specifically exhibited reproducible high β -galactosidase activity. Quantitative β -galactosidase assays, using o-nitrophenyl- β -D-galactoside, were carried out on permeabilized cells, as described previously (Yamada, K., Osawa, H., and Granner, D. K. (1999) FEBS Lett. 460, 41-45; Yamada, K., Printz, R. L., Osawa, H., and Granner, D. K. (1999) Biochem. Biophys. Res. Commun. 261, 614-621 and others). Nucleotide sequences from each positive clone were compared with those entered in the GenBank database using the BLAST sequence search and comparison program.

### Reference example 3: Rapid amplification of cDNA ends (RACE)

To obtain the 5' end of the human ZHX3 cDNA, we employed a 5'-RACE method using human testis marathon-ready cDNA and the Advantage 2 PCR kit. Two gene-specific primers, hZHX3-5RACE-As1 (SEQ ID NO: 34), and hZHX3-5RACE-As2 (SEQ ID NO: 35), were used. The 5'-RACE procedure was carried out according to the manufacturer's recommended protocol. Amplified DNA fragments were subcloned into the pGEM-T Easy vector and their nucleotide sequences determined.

### Reference example 4: Poly(A)+ RNA blot analysis

Human Multiple Tissue Northern Blot and Blot II were hybridized with 0.6-kb EcoR I fragment of the human ZHX3 cDNA, isolated from the pGEM-T Easy hZHX3N plasmid and labelled with [α⁻³²P] dCTP using BcaBest DNA labelling kit. The ExpressHyb hybridization solution was used for prehybridization and hybridization. Prehybridization, hybridization and washing procedures were performed according to the protocol provided by the supplier.

### Reference example 5: Yeast two-hybrid system and liquid β -galactosidase assays

To analyse the heterodimerization domain of ZHX1 with ZHX3, SFY526 yeast strains harbouring pDBD or pDBD-G23 were transformed with various truncated forms of ZHX1 fused to the GAL4 AD, or pACT2. To map the heterodimerization domain of ZHX3 with ZHX1 or the homodimerization domain of ZHX3, SFY526 yeast strains harbouring the pDBD, pDBD-ZHX1 (1-873), or pDBD-G23 were transformed with various truncated forms of ZHX3 fused to the GAL4 AD, or pACT2. In order to examine the interaction domain of ZHX3 with NF-YA, SFY526 yeast strains harbouring pDBD or pYA1-269 were transformed with various truncated forms of ZHX3 fused to the GAL4 AD. For mapping the interacting domain of NF-YA with ZHX3, a SFY526 yeast strain harbouring pAD-ZHX3 (242-488) was transformed with various truncated forms of NF-YA fused to the GAL4 DBD.

These β-galactosidase activities were determined as described previously (Yamada, K., Osawa, H., and Granner, D. K. (1999) FEBS Lett. 460, 41-45; Yamada, K., Printz, R. L., Osawa, H., and Granner, D. K. (1999) Biochem. Biophys. Res. Commun. 261, 614-621; Hirano, S., Yamada, K., Kawata, H., Shou, Z., Mizutani, T., Yazawa, T., Kajitani, T., Sekiguchi, T., Yoshino, M., Shigematsu, Y., Mayumi, M., and Miyamoto, K. (2002) Gene 290, 107-114).

### Reference example 6: GST pull-down assays

The pGST-ZHX1 (1-873) plasmids have been described previously (Hirano, S., Yamada, K., Kawata, H., Shou, Z., Mizutani, T., Yazawa, T., Kajitani, T., Sekiguchi, T., Yoshino, M., Shigematsu, Y, Mayumi, M., and Miyamoto, K. (2002) Gene 290, 107-114). TOPP3 cells were transformed with pGEX-5X-1, pGST-ZHX1 (1-873), pGST-G23 or the pGST-ZHX3 (1-956) fusion-protein expression plasmid. The preparation of the GST fusion protein, ³⁵S-labelling of in vitro-translated ZHX1 and pull-down analysis have been described previously (Yamada, K., Printz, R. L., Osawa, H., and Granner, D. K. (1999) Biochem. Biophys. Res. Commun. 261, 614-621; Hirano, S., Yamada, K., Kawata, H., Shou, Z., Mizutani, T., Yazawa, T., Kajitani, T., Sekiguchi, T., Yoshino, M., Shigematsu, Y, Mayumi, M., and Miyamoto, K. (2002) Gene 290, 107-114). The pDBD-ZHX3 (1-956) plasmid was employed for the preparation of in vitro-translated ³⁵S-labelled ZHX3. Finally, the beads were resuspended in an equal volume of 2xSDS sample buffer and each supernatant was loaded on to an SDS/PAGE gel (10%), along with a prestained molecular-mass marker. The gel was dried and exposed to a FUJIX imaging plate (Kanagawa, Japan). Interaction signals were detected using the FUJIX BAS-2000 image analysing system. The relative purity and amounts of each fusion protein were determined by gel-staining with Coomassie Brilliant Blue R-250.

### Reference example 7 Cell culture and DNA transfections

HEK293 cells were cultured in Dulbecco's modified Eagle's medium supplemented with 10% fetal bovine serum at 37 DEG C in a 5% CO2 incubator.

DNA transfections were carried out using the Lipofectamine Plus reagents. All plasmids used for the transfection were prepared using a Qiagen plasmid kit, followed by CsCl gradient ultracentrifugation. Cells (5x10⁴/well) were inoculated in a 24-well plate on the day prior to transfection. The 5xGAL4-pGL3 Control has been described previously (Tanaka, T., Inazu. T., Yamada, K., Myint, Z., Keng, V W., Inoue, Y., Taniguchi, N., and Noguchi, T. (1999) Biochem. J. 339, 111-117). 5xGAL4-pGL3 Control or pGL3-Control was employed as the reporter plasmid. For the determination of transcriptional activity of ZHX3, 100 ng of a reporter plasmid, 2 ng of the pRL-CMV and the indicated amount of GAL4 DBD-ZHX3 fusion-protein expression plasmid were transfected. The total amount of plasmid (152 ng) was adjusted by the addition of the pSG424, if required. For the analysis of effects of heterodimerization of ZHX3 with ZHX1 on the transcriptional activity of ZHX3, 100 ng of a reporter plasmid, 2 ng of the pRL-CMV, 50 ng of GAL4 DBD fusion-protein expression plasmids, and the indicated amount of the expression plasmid for the dimerization domain of the human ZHX1, pCMV-ZHX1 (272-432), were transfected. The total amount of plasmid (202 ng) was adjusted by the addition of the pcDNA3.1His-C2, if necessary. For observation of the green fluorescent protein (GFP) fusion protein, 300 ng of the indicated GFP plasmid was transfected. Then, 3 h after transfection, the medium was changed. After 48 h the cells were subjected to luciferase assays or observed with a laser microscope (Olympus). Firefly and sea pansy luciferase assays were performed according to the manufacturer's recommended protocol (Promega). Luciferase activities were determined by a Berthold Lumat model LB 9501 (Wildbad, Germany). Firefly luciferase activities (relative light units) were normalized by sea pansy luciferase activities.

### Example 1

In this example, to analyze the molecular mechanism of transcriptional repression by ZHX1 and to examine the issue of whether the human ZHX1 interacts with either a known or a novel transcription factor, the inventors conducted a screening of ZHX1-interacting proteins. An entire coding sequence of the human ZHX1 was fused to the GAL4 DBD and this chimaeric protein was employed as the bait to screen rat liver and granulosa cell cDNA libraries (reference example 2) using the yeast two-hybrid system (reference example 5). Approx. 1.5x10⁷ and 1.1×10⁷ independent clones of each library were screened, and 16 and 25 clones showed reproducible His⁺, Ade⁺ and α -gal-positive properties, respectively. The inventors isolated plasmids that encode the GAL4 AD fusion protein from these clones. After determination of their nucleotide sequences, they were compared with the GenBank database using the BLAST search program. The results were shown in Table 1.

**Table 1**

| **The ZHX1-interacting proteins** | |
|---|---|
| **Protein** | **Number of clone** |
| **BS69 corepressor** | **9** |
| **Nuclear protein, ataxia-telangiectasia-like protein** | **5** |
| **Androgen-induced alsose reductase** | **3** |
| **ATF-IP** | **2** |
| **Spinocerebellar ataxia type I** | **2** |
| **Zyxin** | **2** |
| **Elf-1** | **1** |
| **Eleven-nineteen lysine-rich leukemia gene** | **1** |
| **ZHX1** | **8** |
| **unknown** | **8** |

As shown in Table 1, the BS69 co-repressor, nuclear protein, ataxia-telangiectasia-like protein, androgen-induced aldose reductase, activating transcription factor (ATF)-interacting protein (ATF-IP), spinocerebellar ataxia type I, zyxin, Elf-1, eleven-nineteen lysine-rich leukemia gene and ZHX1 were cloned as known proteins (Hirano, S., Yamada, K., Kawata, H., Shou, Z., Mizutani, T., Yazawa, T., Kajitani, T., Sekiguchi, T., Yoshino, M., Shigematsu, Y., Mayumi, M., and Miyamoto, K. (2002) Gene 290, 107-114 and others). Eight clones encoded unknown proteins. Interestingly, three clones, G23, G58 and L26, encoded both Znf and HD motifs. A detailed nucleotide sequence analysis showed that the nucleotide sequence of G23 was included in that of G58, and that the nucleotide sequence exhibited a similarity to that of partially cloned human KIAA0395 cDNA. The inventors focused on the analysis of these clones in this study. The L26 clone, differing from the KIAA0395, has been denoted ZHX2 and will be reported on in the future.

In order to isolate the 5'-non-coding sequence and the remaining coding region of the human KIAA0395 cDNA, the inventors then employed a 5'-RACE method (reference example 3). Using combinations of gene-specific primers and adaptor primers, a cDNA fragment was obtained by PCR using human testis marathon cDNA as a template. Finally, the size of the full-length cDNA was determined to be 9302 bp.

Very interestingly, the full-length cDNA has an open reading frame of 956 amino acid residues and the deduced amino acid sequence of the protein contains two Cys₂-His₂-type Znf motifs and five HDs as well as ZHX1. The amino acid sequence is shown in SEQ ID NO: 1. Hereafter, the inventors refer to the protein as ZHX3. The name ZHX3 has been submitted to the HUGO Nomenclature Committee with the name zinc-fingers and homeoboxes 3. Figure 1 shows a comparison of deduced amino acid sequences of human ZHX3 with human ZHX1

The human ZHX3 protein has a predicted molecular mass of 104.7 kDa and a pI of 5.68. Whereas pAD-G58 and pAD-G23 encoded amino acid sequence between 114 and 642 and between 242 and 615 of the ZHX3, respectively, KIAA0395 encoded amino acid sequence between 498 and 956 of the human ZHX3. A glutamic-acid-rich region that may act as a transcription regulatory domain existed in the amino acid sequence between 670 and 710 and no putative nuclear-localization signals exist. The similarity in nucleotide sequences in the coding region and amino acid sequences between ZHX3 and ZHX1 were 46.9% and 34.4%, respectively.

Then, the tissue distribution of human ZHX3 mRNA was determined by Northern blot analysis (reference example 4). As shown in Figure 2, human ZHX3 mRNA was detected as multiple bands, 9.4, 7.3, 5.0 and 4.6 kb in length. Since the size of the inventor's cloned insert was 9302 bp, it is almost identical with the full-length transcript. These transcripts were observed in all the tissues examined, although the intensity varied among tissues. This indicates that human ZHX3 mRNA is expressed ubiquitously.

### Example 2

To examine the issue of which domain of ZHX1 is required for interaction with ZHX3 in this example, the inventors conducted mapping of the minimal hetero-dimerization domain between ZHX1 and ZHX3. A yeast strain SFY526 was transformed with the pDBD, which expresses GAL4 DBD alone, or pDBD-G23, which encodes amino acid residues 242-615 of the ZHX3 fused to GAL4 DBD. The two yeast strains were used as the reporter yeasts. pACT2, which expresses GAL4 AD alone or some plasmids, which encode various truncated forms of ZHX1 fused to the GAL4 AD, were employed as the prey plasmids. When a reporter yeast harbouring the pDBD was transformed with these prey plasmids, they showed low β -galactosidase activities (results not shown). In addition, when a reporter yeast harbouring the pDBD-G23 was transformed with the pACT2, pAD-ZHX1 (565-873), pAD-ZHX1 (430-564) or pAD-ZHX1 (345-463), these yeasts also showed low β -galactosidase activities (Figure 3). In contrast, when the yeast was transformed with the pAD-ZHX1 (142-873), pAD-ZHX1 (272-873), pAD-ZHX1 (272-564) or pAD-ZHX1 (272-432), high β -galactosidase activities were observed. pAD-ZHX1 (272-432) encodes amino acid residues between 272 and 432 of ZHX1.

The inventors then determined the issue of which domain of ZHX3 is required for an interaction with ZHX1. Yeast strain SFY526 was transformed with pDBD-ZHX1 (1-873), which encodes an entire coding sequence of the ZHX1 fused to GAL4 DBD, or pDBD. The two yeast strains were used as the reporter yeasts. The pACT2 or some plasmids encoding various truncated forms of ZHX3 fused to the GAL4 AD were employed as prey plasmids (Figure 4). When a reporter yeast harbouring the pDBD was transformed with these prey plasmids, they showed low β -galactosidase activities (results not shown).

These yeasts showed high β -galactosidase activities only when a reporter yeast harbouring pDBD-ZHX1 (1-873) was transformed with pAD-G23 or pAD-ZHX3 (242-488). pAD-ZHX3 (242-488) encodes the amino acid residues between 242 and 488 of ZHX3. These results reveal that ZHX1 and ZHX3 form a heterodimer via each region including HD1.

The inventors also carried out in vitro GST pull-down assays (reference example 6) to verify the specific interaction between ZHX1 and ZHX3. The inventors employed four plasmids, pGEX-5X-1, which expresses GST alone, pGST-G23, which encodes the amino acids 242-615 of human ZHX3 fused to GST, pGST-ZHX3 (1-956), which expresses the entire coding region of human ZHX3 protein fused to GST, and pGST-ZHX1 (1-873), which expresses the entire coding region of human ZHX1 protein fused to GST. These proteins were expressed in *Escherichia coli* and immobilized on to glutathione-Sepharose beads. The *in vitro*-translated, ³⁵S-labelled full-length of human ZHX1 was found to bind to GST-G23 and GST-ZHX3 (1-956) but not to GST alone (Figure 5, lanes 3 and 6). In addition, the in vitro-translated, ³⁵S-labelled full-length human ZHX3 was found to bind to GST-ZHX1 (1-873) but not to GST alone (Figure 5, lane 9). In contrast, an unprogrammed reticulocyte lysate failed to bind to any of the proteins (results not shown).

These results indicate that ZHX1 is able to form a heterodimer with ZHX3 both *in vivo* and *in vitro.*

### Example 3

Since ZHX1 forms a homodimer, the inventors conducted a mapping of the minimal homodimerization domain of ZHX3, to investigate formation of a homodimer for ZHX3 using the yeast two-hybrid system (reference example 5). Two SFY526 yeast strains harbouring pDBD or pDBD-G23 were used as the reporter yeasts. The inventors prepared various prey plasmids, pACT2, pAD-G23, pAD-ZHX3 (242-488), pAD-ZHX3 (303-364) and pAD-ZHX3 (498-903). These plasmids were transformed into the reporter yeasts and β -galactosidase activity was determined in each case (Figure 6). When the reporter yeast harbouring pDBD was transformed with the plasmids, they showed very low β -galactosidase activities (results not shown). In addition, when a reporter yeast harbouring pDBD-G23 was transformed with pACT2, pAD-ZHX3 (303-364) and pAD-ZHX3 (498-903), very low β -galactosidase activity was also detected. In contrast, the yeast transformed with pAD-G23 or pAD-ZHX3 (242-488) expressed high β -galactosidase activities. These results indicate that ZHX3 is able to form a homodimer via the region between residues 242 and 488.

The inventors then carried out in vitro GST pull-down assays to verify the homodimerization of ZHX3. The inventors employed two plasmids, pGEX-5X-1 and pGST-ZHX3 (1-956). These proteins were expressed in *E*. *coli* and immobilized on to glutathione-Sepharose beads. The *in vitro*-translated, ³⁵S-labelled full-length human ZHX3 was found to bind to GST-ZHX3 (1-956) but not to GST alone (Figure 7). In contrast, an unprogrammed reticulocyte lysate failed to bind to any of the proteins (results not shown). These results indicate that ZHX3 is able to form a homodimer *in vivo* and *in vitro*.

### Example 4

In this example, the inventors investigated the issue whether ZHX3 also interacts with the AD of the NF-YA. Human ZHX1 was originally cloned as a protein that interacts with NF-YA (Yamada, K., Printz, R. L., Osawa, H., and Granner, D. K. (1999) Biochem. Biophys. Res. Commun. 261, 614-621). The inventors examined the interaction of ZHX3 with NF-YA using the yeast two-hybrid system (reference example 5). The inventors used two reporter-yeast strains, which are transformed with pDBD or pYA1-269. pYA1-269 expresses the AD of the NF-YA fused to the GAL4 DBD. pACT2, pAD-ZHX3 (1-956), pAD-G23, pAD-ZHX3 (242-488), pAD-ZHX3 (303-364) and pAD-ZHX3 (498-903) were transformed into the reporter yeast strains and their β -galactosidase activities determined. When a reporter yeast harbouring the pDBD was transformed with these plasmids, their β -galactosidase activities were found to be quite low (results not shown). As shown in the middle of Figure 8, when a reporter yeast harbouring pYA1-269 was transformed with pACT2, pAD-ZHX3 (303-364) or pAD-ZHX3 (498-903), their β -galactosidase activities were also low. However, when the yeast was transformed with pAD-ZHX3 (1-956), pAD-G23 or pAD-ZHX3 (242-488), high levels of β -galactosidase activity were detected. These results indicate that ZHX3 interacts with the AD of the NF-YA, and that the amino acid sequence between residues 242 and 488 is essential for this interaction.

The inventors next identified the minimal interaction domain of NF-YA with ZHX3 using the yeast two-hybrid system (reference example 5). As shown in Figure 9, the AD of NF-YA consists of a glutamine-rich and a serine/threonine-rich domain. The SFY526 yeast strain harbouring pAD-ZHX3 (242-488) was used as a reporter yeast. Various plasmids expressing truncated forms of the NF-YA fused to the GAL4 DBD were transformed in the yeast and their β -galactosidase activities determined. As a result, only yeasts harbouring the pYA1-269 or pYA141-269, both of which contain amino acids 141-269 of NF-YA, showed a high level of β -galactosidase activity. These results indicate that a serine/threonine-rich AD of NF-YA represents the minimal interaction domain with ZHX3.

### Example 5

In this example, to confirm that ZHX3 is a transcriptional repressor, the inventors determined the transcriptional role of ZHX3 using a mammalian one-hybrid system (reference example 7). The 5xGAL4-pGL3 Control plasmid, in which five copies of the GAL4-binding site had been inserted upstream of the simian virus 40 (SV40) promoter of pGL3-Control, was employed as a reporter plasmid. Two effector plasmids, pSG424, which expresses GAL4 DBD alone, and pGAL4-ZHX3 (1-956), which expresses the entire coding region of human ZHX3 fused to the C-terminus of the GAL4 DBD, were prepared. As shown in Figures 10 and 11, when 5xGAL4-pGL3 Control and various amounts of pGAL4-ZHX3 (1-956) were co-transfected into HEK293 cells, the luciferase activity was decreased in a dose-dependent manner. The maximal inhibition was obtained with 50 ng of the pGAL4-ZHX1 (1-956). In contrast, when the pGL3-Control lacking five copies of the GAL4-binding sites was transfected with the pSG424 or pGAL4-ZHX3 (1-956), the luciferase activities remained unchanged (Figure 10). These results show that the GAL4-ZHX3 fusion protein decreases luciferase activity in a GAL4-binding-site-dependent manner, indicating that ZHX3 acts as a transcriptional repressor.

The inventors then examined the issue of whether heterodimerization of ZHX3 with ZHX1 is required for its transcriptional repressor activity. The inventors prepared the pCMV ZHX1 (272-432), in which amino acids 272-423 of ZHX1 are expressed. Although this region corresponds to the dimerization domain of ZHX1 with ZHX3, it does not contain the repressor domain of ZHX1 (Yamada, K., Kawata, H., Matsuura, K., Shou, Z., Hirano, S., Mizutani, T., Yazawa, T., Yoshino, M., Sekiguchi, T., Kajitani, T., and Miyamoto. K. (2002) Biochem. Biophys. Res. Comm. 279, 368-374). Therefore, the overexpression of this protein functions as a dominant-negative form of ZHX1. When the plasmid was co-transfected in the above assay system, the luciferase activity was increased in a dose-dependent manner (Figure 11). In contrast, co-transfection of the pcDNA3.1HisC-2 had no effect on luciferase activity. These results suggest that heterodimerization of ZHX3 with ZHX1 is a prerequisite for repressor activity.

Finally, to determine the minimal repressor domain of ZHX3, 5xGAL4-pGL3 Control was transfected with various plasmids, pGAL4-ZHX3 (1-145), pGAL4-ZHX3 (146-303), pGAL4-ZHX3 (303-555) or pGAL4-ZHX3 (498-903) (Figure 12). Only pGAL4-ZHX3 (303-555), which expresses amino acids 303-555, led to a decrease in luciferase activity.

For a more detailed analysis, the inventors prepared the effector plasmids pGAL4-ZHX3 (303-502), pGAL4-ZHX3 (303-364) and pGAL4-ZHX3 (364-502). Only when pGAL4-ZHX3 (303-502) was transfected with the reporter plasmid did the luciferase activity decrease (Figure 12). These results show that the amino acid sequence between residues 303 and 502 of ZHX3 are essential for repressor activity.

### Example 6

To examine the subcellular localization of the ZHX3 protein, the inventors determined the subcellular localization of ZHX3 and mapped NLSs, employing the GFP-ZHX3 fusion-protein expression system. Various truncated forms of ZHX3 fused to GFP were prepared. These plasmids were transfected into HEK293 cells and the subcellular localization of the GFP fusion proteins observed. pEGFP-C1E1, encoding GFP protein alone, was observed in the whole cell (Figure 13). In contrast, GFP-ZHX3 (1-956), in which full-length ZHX3 was fused to the C-terminus of GFP, was localized in the nuclei. To determine the NLS of ZHX3, various plasmids were transfected. When pGFP-ZHX3 (1-303), pGFP-ZHX3 (303-555) and pGFP-ZHX3 (555-956) were transfected, both pGFP-ZHX3 (1-303) and pGFP-ZHX3 (303-555) were located in the nuclei. In contrast, when pGFP-ZHX3 (555-956) was transfected, the protein was localized outside of the nuclei. These results suggest that ZHX3 contains two NLS and a nuclear export signal (NES). To map the minimal NLSs, various plasmids were constructed. Only when three plasmids, pGFP-ZHX3 (1-107), pGFP-ZHX3 (364-555) and pGFP-ZHX3 (497-555), were transfected, the nuclear localization of ZHX3 was observed. These results show that ZHX3 is able to localize in the nuclei as a GFP fusion protein, and that two NLSs of ZHX3 are located in amino acids 1-107 and 497-555.

In the above examples, the inventors conducted a search for ZHX1-interacting proteins, mainly by analysing a novel transcriptional repressor of them, ZHX3, and mapped its functional domains. The minimal functional domains of ZHX3 are summarized in Figure 14. ZHX3 as well as ZHX1 contains two Znf motifs and five HDs, forms a homodimer, interacts with the AD of NF-YA, and is localized in the nucleus. In addition, ZHX3 mRNA is expressed ubiquitously. From these findings, the inventors conclude that both ZHX1 and ZHX3 are members of the same family, namely the ZHX family.

While the similarity of the entire amino acid sequences of ZHX1 and ZHX3 was 34.4%, the two Znf motifs and five HDs were highly conserved. Similarities in the amino acid sequences of Znf1, Znf2, HD1, HD2, HD3, HD4 and HD5 between ZHX1 and ZHX3 were 50.0, 45.5, 61.7, 50.0, 53.3, 43.3 and 33.3%, respectively. The HD4 showed a much lower similarity than the other domains. A unique glutamic-acid-rich acidic region is located in the amino acid sequence between residues 670 and 710 of ZHX3 (Figures 1 and 9). Generally, the Znf motif, the HD and the acidic region are responsible for the functional properties of the transcription factor. For example, both the Znf motif and HD, which consist of 60 amino acids, are required for binding to the cognate DNA sequence, the glutamic-acid-rich regions are involved in transcriptional activity, and the basic region is the DBD or NLS (Gehring, W. J., Affolter, M., and Burglin, T. (1994) Annu. Rev. Biochem. 63 , 487-526 and others).

ZHX3 not only forms a heterodimer with ZHX1 but also forms a homodimer. Amino acids 242-488 of ZHX3 are necessary and sufficient for these dimerizations (Figures 3 -7). A minimal domain for the homo- and hetero-dimerization of ZHX1 with ZHX3 was mapped to amino acids 272-432 of ZHX1 (Figures 3 -5). These regions include the HD1 but HD1 alone failed to dimerize (Figures 3-9). A more extensive region including HD1 is required for dimerization. Furthermore, both ZHX3 and ZHX1 interact with the AD of NF-YA; the former interacts with a serine/threonine-rich AD and the latter with a glutamine-rich AD of the NF-YA, respectively (Figures 8 and 9). An interaction domain of ZHX3 with the AD of NF-YA was mapped to the same region as the dimerization domain of ZHX3 (Figures 8 and 9). In contrast, the amino acid sequence between 272 and 564, which contains the HD1-HD2 region of human ZHX1, is required for its interaction (Yamada, K., Osawa, H., and Granner, D. K. (1999) FEBS Lett. 460, 41-45). The issue of whether a heterodimer complex of ZHX1 with ZHX3 interacts with different ADs of NF-YA remains to be determined.

ZHX3 is a transcriptional repressor (Figures 10-12). The minimal repressor domain of ZHX3 is mapped to an overlapping region with both dimerization and interaction domains. Interestingly, the overexpression of the heterodimerization domain of ZHX1, which is not responsible for repressor activity, led to a decrease in the repressor activity of ZHX3 (Figure 11). This raises the possibility that ZHX3 itself has no repressor activity and that the observed activity is dependent upon the repressor activity of a dimerization partner, ZHX1, in which the repressor domain is located in the C-terminus of the acidic region (Yamada, K., Kawata, H., Matsuura, K., Shou, Z., Hirano, S., Mizutani, T., Yazawa, T., Yoshino, M., Sekiguchi, T., Kajitani, T., and Miyamoto. K. (2002) Biochem. Biophys. Res. Comm. 279, 368-374). However, it cannot be ruled out that the dimerization domain of ZHX3 has bona fide repressor activity. It is unclear whether ZHX3 is a DNA-binding protein or not. As a result, it appears that a region of ZHX3 including the HD1 region is a pleiotropic domain; a homo- and hetero-dimerization domain with ZHX1, an interaction domain with the AD of NF-YA, and a repressor domain.

Regions of transcriptional regulation interact with cofactors to function as transcriptional repressors (Hu, X., and Lazar, M. A. (2000) Trends Endocrinol. Metab. 11, 6-10 and others). These cofactors include mSin3A/B, histone deacetylases and the nuclear co-repressor (N-CoR)/silencing mediator of receptor transcription. As ZHX1-interacting proteins the inventors cloned two co-repressors, BS69 and ATF-IP (Table 1) (Hateboer, G., Gennissen, A., Ramos, Y. F. M., Kerkhoven, R. M., Sonntag-Buck, V, Stunnenberg, H. G., and Bernards. R. (1995) EMBO J. 14, 3159-3169 and others). BS69 was first identified as a protein that interacts directly with the AD of the 289R adenovirus type 5 E1A protein. It has been also reported that BS69 mediates repression, at least in part, through an interaction of the MYND domain of BS69 with the co-repressor N-CoR (Masselink, H., and Bernards, R. (2000) Oncogene 19, 1538-1546). In contrast, ATF-IP interacts with several components of the basal transcription machinery (TFIIE and TFIIH), including RNA polymerase II holoenzyme (DeGraeve, F., Bahr, A., Chatton, B., and Kedinger, C. (2000) Oncogene 10, 1807-1819). When ZHX1 and ZHX3 act as a transcriptional repressor, it could interact with these co-repressors, thus repressing gene transcription.

Furthermore, both ZHX1 and ZHX3 are NF-YA-interacting proteins. It has been reported that NF-Y is associated with co-activators, p300 and p300/cAMP response element-binding protein-binding protein-associated factor (P/CAF) (Mantovani, R. (1999) Gene 239, 15-27). In particular, P/CAF with histone acetyltransferase activity interacts with the NF-YA to form a transcriptionally active NF-Y complex (Mantovani, R. (1999) Gene 239, 15-27). Therefore, it is likely that combinations of interactions among ZHX1, ZHX3 and NF-YA affect the transcriptional activity of NF-Y. For example, either ZHX1 or ZHX3, or both, may enhance or interfere with the association of P/CAF with the NF-Y, thus regulating NF-Y activity. In addition, the transcriptional repressor, a member of the ZHX family, and a co-repressor are able to directly associate with the NF-YA, thus inhibiting NF-Y activity. In any case, it is possible that the ZHX proteins participate in the regulation of a number of NF-Y-regulatable genes.

Although ZHX3 mRNA is expressed ubiquitously, it was found to be expressed more highly in skeletal muscle, kidney and testis (Figure 2). The size of ZHX3 mRNA varied as determined by Northern blot analysis. The cloned insert contains 9302 bp and the size is the same as the largest transcript of ZHX3. When a search for the human ZHX3 gene was conducted using the database compiled by the Human Genome Project, it was found to be located in chromosome 20q. This suggests that the ZHX3 gene exists as a single copy per haploid human genome. The nucleotide sequence of ZHX3 cDNA revealed that multiple polyadenylation signals exist in the 3'-non-coding region. Therefore, it is likely that smaller mRNAs might be produced by the use of different polyadenylation signals from a single gene rather than by the existence of other ZHX3-related mRNAs.

When the entire coding region of ZHX3 was fused to the C-terminal end of the GFP, it became localized in the nuclei (Figure 13). There were two NLSs of ZHX3, amino acids 1-107 and 497-555. On the other hand, amino acids 498-956 of ZHX3 fused to GFP become exclusively localized not in the entire cell but external to the nucleus. GFP alone or the GFP-ZHX1 fusion protein lacking the NLS was localized to entire cells (Figure 13). This indicates that this region of ZHX3 contains a nuclear-export signal. Therefore, ZHX3 is a more complicated protein that contains two NLSs and a nuclear-export signal in one molecule. In many other proteins, including ZHX1, it has been reported that the NLS was mapped to a cluster of basic amino acid residues (Yamada, K., Kawata, H., Matsuura, K., Shou, Z., Hirano, S., Mizutani, T., Yazawa, T., Yoshino, M., Sekiguchi, T., Kajitani, T., and Miyamoto. K. (2002) Biochem. Biophys. Res. Comm. 279, 368-374 and others). This region is associated with nuclear-importing proteins such as importin α and is then translocated from the cytoplasm to the nuclei (Kaffman, A., and O'Shea, E. K. (1999) Annu. Rev. Cell Dev. Biol. 15, 291-339). However, ZHX3 may associate with other molecules in order to be translocated to the nuclei, since the two NLSs of ZHX3 are not located in the basic region and do not exhibit any similarity with previously reported NLS.

## Claims

1. A protein or a peptide of any of (a) to (d) having a transcriptional repressor activity for use as a medicament to treat hepatoma:
(a) a protein or a peptide having an amino acid sequence of SEQ ID NO: 1;
(b) a protein or a peptide having an amino acid sequence with at least 85% similarity to the amino acid sequence of SEQ ID NO: 1;
(c) a protein or a peptide comprising the functional domain consisting of amino acids 303-502 of the amino acid sequence of SEQ ID NO: 1;
(d) a protein or a peptide comprising an amino acid sequence with at least 85% similarity to the functional domain consisting of amino acids 303-502 of SEQ ID NO: 1.

2. The protein or peptide of Claim 1, wherein said protein or peptide is a repressor of the transcription of type II hexokinase gene or pyruvate kinase M gene.

3. The use of a screening agent to screen drug agents with transcriptional repressor activity for treating hepatoma, wherein the screening agent comprise an antibody specific to a protein or a peptide of any of (a) to (d) as an effective component:
(a) a protein or a peptide having an amino acid sequence of SEQ ID NO: 1;
(b) a protein or a peptide having an amino acid sequence with at least 85% similarity to the amino acid sequence of SEQ ID NO: 1, and having a transcriptional repressor activity;
(c) a protein or a peptide comprising the functional domain consisting of amino acids 303-502 of the amino acid sequence of SEQ ID NO:
(d) a protein or a peptide comprising an amino acid sequence with at least 85% similarity to the functional domain consisting of amino acids 303-502 of SEQ ID NO: 1 and having a transcriptional repressor activity.

## Patentansprüche

1. Protein oder Peptid nach einem von (a) bis (d) mit einer transkriptionellen Repressor-Aktivität zur Verwendung als Medikament zur Hepatom-Behandlung:
(a) Protein oder Peptid mit einer Aminosäuresequenz von SEQ ID NO: 1;
(b) Protein oder Peptid mit einer Aminosäuresequenz mit mindestens 85 % Ähnlichkeit zu der Aminosäuresequenz von SEQ ID NO: 1;
(c) Protein oder Peptid, umfassend die funktionelle Domäne, bestehend aus den Aminosäuren 303-502 der Aminosäuresequenz von SEQ ID NO: 1;
(d) Protein oder Peptid, umfassend eine Aminosäuresequenz mit mindestens 85 % Ähnlichkeit zu der funktionellen Domäne, bestehend aus den Aminosäuren 303-502 von SEQ ID NO: 1.

2. Protein oder Peptid nach Anspruch 1, wobei das Protein oder Peptid ein Repressor der Transkription des Typ-II-Hexogenasegens oder des Pyruvatkinase M-Gens ist.

3. Die Verwendung eines Screening-Mittels zum Screening von Arzneimitteln mit transkriptioneller Repressoraktivität zur Hepatom-Behandlung, wobei das Screening-Mittel einen Antikörper, der für ein Protein oder ein Peptid nach einem von (a) bis (d) spezifisch ist, als eine wirksame Komponente umfasst:
(a) Protein oder Peptid mit einer Aminosäuresequenz von SEQ ID NO: 1;
(b) Protein oder Peptid mit einer Aminosäuresequenz mit mindestens 85 % Ähnlichkeit zu der Aminosäuresequenz von SEQ ID NO: 1, und mit einer transkriptionellen Repressor-Aktivität;
(c) Protein oder Peptid, umfassend die funktionelle Domäne, bestehend aus den Aminosäuren 303-502 der Aminosäuresequenz von SEQ ID NO: 1;
(d) Protein oder Peptid, umfassend eine Aminosäuresequenz mit mindestens 85 % Ähnlichkeit zu der funktionellen Domäne, bestehend aus den Aminosäuren 303-502 von SEQ ID NO: 1, und mit einer transkriptionellen Repressor-Aktivität.

## Revendications

1. Protéine ou peptide selon l'un quelconque de (a) à (d) ayant une activité de répresseur de la transcription, destiné(e) à être utilisé(e) en tant que médicament pour traiter un hépatome :
(a) une protéine ou un peptide ayant une séquence d'acides aminés de SEQ ID NO: 1;
(b) une protéine ou un peptide ayant une séquence d'acides aminés présentant au moins 85 % de similarité avec la séquence d'acides aminés de SEQ ID NO: 1 ;
(c) une protéine ou un peptide comprenant le domaine fonctionnel consistant en les acides aminés 303-502 de la séquence d'acides aminés de SEQ ID NO: 1 ;
(d) une protéine ou un peptide comprenant une séquence d'acides aminés ayant au moins 85 % de similarité avec le domaine fonctionnel consistant en les acides aminés 303-502 de SEQ ID NO: 1.

2. Protéine ou peptide selon la revendication 1, où ladite protéine ou ledit peptide est un répresseur de la transcription du gène de l'hexokinase de type II ou du gène de la pyruvate kinase M.

3. Utilisation d'un agent de criblage pour cribler des agents médicamenteux ayant une activité de répresseur de la transcription pour traiter un hépatome, où l'agent de criblage comprend un anticorps spécifique pour une protéine ou un peptide selon l'un quelconque de (a) à (d) en tant que composant efficace :
(a) une protéine ou un peptide ayant une séquence d'acides aminés de SEQ ID NO: 1;
(b) une protéine ou un peptide ayant une séquence d'acides aminés présentant au moins 85 % de similarité avec la séquence d'acides aminés de SEQ ID NO: 1, et ayant une activité de répresseur de la transcription ;
(c) une protéine ou un peptide comprenant le domaine fonctionnel consistant en les acides aminés 303-502 de la séquence d'acides aminés de SEQ ID NO: 1 ;
(d) une protéine ou un peptide comprenant une séquence d'acides aminés ayant au moins 85 % de similarité avec le domaine fonctionnel consistant en les acides aminés 303-502 de SEQ ID NO: 1 et ayant une activité de répresseur de la transcription.


### SEQUENCE LISTING

<110> Japan Science and Technology Agency
<120> Transcriptional regulator ZHX3
<130> FS03-323PCT
<160> 35
<170> PatentIn version 3.1
<210> 1
   <211> 956
   <212> PRT
   <213> Homo sapiens
<400> 1
   Met Ala Ser Lys Arg Lys Ser Thr Thr Pro Cys Met Ile Pro Val Lys
   1 5 10 15
   Thr Val Val Leu Gln Asp Ala Ser Met Glu Ala Gln Pro Ala Glu Thr
   20 25 30
   Leu Pro Glu Gly Pro Gln Gln Asp Leu Pro Pro Glu Ala Ser Ala Ala
   35 40 45
   Ser Ser Glu Ala Ala Gln Asn Pro Ser Ser Thr Asp Gly Ser Thr Leu
   50 55 60
   Ala Asn Gly His Arg Ser Thr Leu Asp Gly Tyr Leu Tyr Ser Cys Lys
   65 70 75 80
   Tyr Cys Asp Phe Arg Ser His Asp Met Thr Gln Phe Val Gly His Met
   85 90 95
   Asn Ser Glu His Thr Asp Phe Asn Lys Asp Pro Thr Phe Val Cys Ser
   100 105 110
   Gly Cys Ser Phe Leu Ala Lys Thr Pro Glu Gly Leu Ser Leu His Asn
   115 120 125
   Ala Thr Cys His Ser Gly Glu Ala Ser Phe Val Trp Asn Val Ala Lys
   130 135 140
   Pro Asp Asn His Val Val Val Glu Gln Ser Ile Pro Glu Ser Thr Ser
   145 150 155 160
   Thr Pro Asp Leu Ala Gly Glu Pro Ser Ala Glu Gly Ala Asp Gly Gln
   165 170 175
   Ala Glu Ile Ile Ile Thr Lys Thr Pro Ile Met Lys Ile Met Lys Gly
   180 185 190
   Lys Ala Glu Ala Lys Lys Ile His Thr Leu Lys Glu Asn Val Pro Ser
   195 200 205
   Gln Pro Val Gly Glu Ala Leu Pro Lys Leu Ser Thr Gly Glu Met Glu
   210 215 220
   Val Arg Glu Gly Asp His Ser Phe Ile Asn Gly Ala Val Pro Val Ser
   225 230 235 240
   Gln Ala Ser Ala Ser Ser Ala Lys Asn Pro His Ala Ala Asn Gly Pro
   245 250 255
   Leu Ile Gly Thr Val Pro Val Leu Pro Ala Gly Ile Ala Gln Phe Leu
   260 265 270
   Ser Leu Gln Gln Gln Pro Pro Val His Ala Gln His His Val His Gln
   275 280 285
   Pro Leu Pro Thr Ala Lys Ala Leu Pro Lys Val Met Ile Pro Leu Ser
   290 295 300
   Ser Ile Pro Thr Tyr Asn Ala Ala Met Asp Ser Asn Ser Phe Leu Lys
   305 310 315 320
   Asn Ser Phe His Lys Phe Pro Tyr Pro Thr Lys Ala Glu Leu Cys Tyr
   325 330 335
   Leu Thr Val Val Thr Lys Tyr Pro Glu Glu Gln Leu Lys Ile Trp Phe
   340 345 350
   Thr Ala Gln Arg Leu Lys Gln Gly Ile Ser Trp Ser Pro Glu Glu Ile
   355 360 365
   Glu Asp Ala Arg Lys Lys Met Phe Asn Thr Val Ile Gln Ser Val Pro
   370 375 380
   Gln Pro Thr Ile Thr Val Leu Asn Thr Pro Leu Val Ala Ser Ala Gly
   385 390 395 400
   Asn Val Gln His Leu Ile Gln Ala Ala Leu Pro Gly His Val Val Gly
   405 410 415
   Gln Pro Glu Gly Thr Gly Gly Gly Leu Leu Val Thr Gln Pro Leu Met
   420 425 430
   Ala Asn Gly Leu Gln Ala Thr Ser Ser Pro Leu Pro Leu Thr Val Thr
   435 440 445
   Ser Val Pro Lys Gln Pro Gly Val Ala Pro Ile Asn Thr Val Cys Ser
   450 455 460
   Asn Thr Thr Ser Ala Val Lys Val Val Asn Ala Ala Gln Ser Leu Leu
   465 470 475 480
   Thr Ala Cys Pro Ser Ile Thr Ser Gln Ala Phe Leu Asp Ala Ser Ile
   485 490 495
   Tyr Lys Asn Lys Lys Ser His Glu Gln Leu Ser Ala Leu Lys Gly Ser
   500 505 510
   Phe Cys Arg Asn Gln Phe Pro Gly Gln Ser Glu Val Glu His Leu Thr
   515 520 525
   Lys Val Thr Gly Leu Ser Thr Arg Glu Val Arg Lys Trp Phe Ser Asp
   530 535 540
   Arg Arg Tyr His Cys Arg Asn Leu Lys Gly Ser Arg Ala Met Ile Pro
   545 550 555 560
   Gly Asp His Ser Ser Ile Ile Ile Asp Ser Val Pro Glu Val Ser Phe
   565 570 575
   Ser Pro Ser Ser Lys Val Pro Glu Val Thr Cys Ile Pro Thr Thr Ala
   580 585 590
   Thr Leu Ala Thr His Pro Ser Ala Lys Arg Gln Ser Trp His Gln Thr
   595 600 605
   Pro Asp Phe Thr Pro Thr Lys Tyr Lys Glu Arg Ala Pro Glu Gln Leu
   610 615 620
   Arg Ala Leu Glu Ser Ser Phe Ala Gln Asn Pro Leu Pro Leu Asp Glu
   625 630 635 640
   Glu Leu Asp Arg Leu Arg Ser Glu Thr Lys Met Thr Arg Arg Glu Ile
   645 650 655
   Asp Ser Trp Phe Ser Glu Arg Arg Lys Lys Val Asn Ala Glu Glu Thr
   660 665 670
   Lys Lys Ala Glu Glu Asn Ala Ser Gln Glu Glu Glu Glu Ala Ala Glu
   675 680 685
   Asp Glu Gly Gly Glu Glu Asp Leu Ala Ser Glu Leu Arg Val Ser Gly
   690 695 700
   Glu Asn Gly Ser Leu Glu Met Pro Ser Ser His Ile Leu Ala Glu Arg
   705 710 715 720
   Lys Val Ser Pro Ile Lys Ile Asn Leu Lys Asn Leu Arg Val Thr Glu
   725 730 735
   Ala Asn Gly Arg Asn Glu Ile Pro Gly Leu Gly Ala Cys Asp Pro Glu
   740 745 750
   Asp Asp Glu Ser Asn Lys Leu Ala Glu Gln Leu Pro Gly Lys Val Ser
   755 760 765
   Cys Lys Lys Thr Ala Gln Gln Arg His Leu Leu Arg Gln Leu Phe Val
   770 775 780
   Gln Thr Gln Trp Pro Ser Asn Gln Asp Tyr Asp Ser Ile Met Ala Gln
   785 790 795 800
   Thr Gly Leu Pro Arg Pro Glu Val Val Arg Trp Phe Gly Asp Ser Arg
   805 810 815
   Tyr Ala Leu Lys Asn Gly Gln Leu Lys Trp Tyr Glu Asp Tyr Lys Arg
   820 825 830
   Gly Asn Phe Pro Pro Gly Leu Leu Val Ile Ala Pro Gly Asn Arg Glu
   835 840 845
   Leu Leu Gln Asp Tyr Tyr Met Thr His Lys Met Leu Tyr Glu Glu Asp
   850 855 860
   Leu Gln Asn Leu Cys Asp Lys Thr Gln Met Ser Ser Gln Gln Val Lys
   865 870 875 880
   Gln Trp Phe Ala Glu Lys Met Gly Glu Glu Thr Arg Ala Val Ala Asp
   885 890 895
   Thr Gly Ser Glu Asp Gln Gly Pro Gly Thr Gly Glu Leu Thr Ala Val
   900 905 910
   His Lys Gly Met Gly Asp Thr Tyr Ser Glu Val Ser Glu Asn Ser Glu
   915 920 925
   Ser Trp Glu Pro Arg Val Pro Glu Ala Ser Ser Glu Pro Phe Asp Thr
   930 935 940
   Ser Ser Pro Gln Ala Gly Arg Gln Leu Glu Thr Asp
   945 950 955
<210> 2
   <211> 522
   <212> PRT
   <213> Rattus norvegicus
<400> 2
   Cys Ser Phe Leu Ala Lys Thr Pro Glu Gly Leu Ser Leu His Asn Ala
   1 5 10 15
   Lys Cys His Ser Gly Glu Ala Ser Phe Leu Trp Asn Val Thr Lys Pro
   20 25 30
   Asp Asn His Val Val Val Glu Gln Ser Val Pro Glu Asn Ala Ser Ser
   35 40 45
   Ser Val Leu Ala Gly Glu Ser Thr Glu Gly Thr Glu Ile Ile Ile Thr
   50 55 60
   Lys Thr Pro Ile Met Lys Ile Met Lys Gly Lys Ala Glu Ala Lys Lys
   65 70 75 80
   Ile His Met Leu Lys Glu Asn Ala Pro Thr Gln Pro Gly Gly Glu Ala
   85 90 95
   Leu Pro Lys Pro Leu Ala Gly Glu Thr Glu Gly Lys Glu Gly Asp His
   100 105 110
   Thr Phe Ile Asn Gly Ala Thr Pro Val Ser Gln Ala Ser Ala Asn Ser
   115 120 125
   Thr Lys Pro Pro His Thr Ala Asn Gly Pro Leu Ile Gly Thr Val Pro
   130 135 140
   Val Leu Pro Ala Gly Ile Ala Gln Phe Leu Ser Leu Gln Gln Pro Thr
   145 150 155 160
   Val His Pro Gln His His Pro His Gln Pro Leu Pro Thr Ser Lys Ala
   165 170 175
   Leu Pro Lys Val Met Ile Pro Leu Ser Ser Ile Pro Thr Tyr Asn Ala
   180 185 190
   Ala Met Asp Ser Asn Ser Phe Leu Lys Asn Ser Phe His Lys Phe Pro
   195 200 205
   Tyr Pro Thr Lys Ala Glu Leu Cys Tyr Leu Thr Val Val Thr Lys Tyr
   210 215 220
   Pro Glu Glu Gln Leu Lys Ile Trp Phe Thr Ala Gln Arg Leu Lys Gln
   225 230 235 240
   Gly Ile Ser Trp Ser Pro Glu Glu Ile Glu Asp Ala Arg Lys Lys Met
   245 250 255
   Phe Asn Thr Val Ile Gln Ser Val Pro Gln Pro Thr Ile Thr Val Leu
   260 265 270
   Asn Thr Pro Leu Val Ala Ser Ala Gly Asn Val Gln His Leu Ile Gln
   275 280 285
   Ala Ala Leu Pro Gly His Ala Val Gly Gln Pro Glu Gly Thr Ala Gly
   290 295 300
   Gly Leu Leu Val Thr Gln Pro Leu Met Ala Asn Gly Leu Gln Ala Ser
   305 310 315 320
   Ser Ser Ser Leu Pro Leu Thr Thr Ala Ser Val Pro Lys Pro Thr Ala
   325 330 335
   Ala Pro Ile Asn Thr Val Cys Ser Asn Thr Thr Ser Ala Val Lys Val
   340 345 350
   Val Asn Ala Ala Gln Ser Leu Leu Thr Ala Cys Pro Ser Ile Thr Ser
   355 360 365
   Gln Ala Phe Leu Asp Ala Asn Ile Tyr Lys Asn Lys Lys Ser His Glu
   370 375 380
   Gln Leu Ser Ala Leu Lys Gly Ser Phe Cys Arg Asn Gln Phe Pro Gly
   385 390 395 400
   Gln Ser Glu Val Glu His Leu Thr Lys Val Thr Gly Leu Ser Thr Arg
   405 410 415
   Glu Val Arg Lys Trp Phe Ser Asp Arg Arg Tyr His Cys Arg Asn Leu
   420 425 430
   Lys Gly Thr Arg Ala Met Val Pro Gly Glu His Gly Ser Val Leu Ile
   435 440 445
   Asp Ser Val Pro Glu Val Pro Phe Pro Leu Ser Ser Lys Val Pro Glu
   450 455 460
   Val Pro Cys Val Pro Thr Ala Thr Ser Leu Val Ser His Pro Ala Thr
   465 470 475 480
   Lys Arg Gln Ser Trp His Gln Thr Pro Asp Phe Thr Pro Thr Lys Tyr
   485 490 495
   Lys Glu Arg Ala Pro Glu Gln Leu Arg Val Leu Glu Ser Ser Phe Ala
   500 505 510
   Gln Asn Pro Leu Pro Pro Glu Glu Glu Leu
   515 520
<210> 3
   <211> 19
   <212> DNA
   <213> Artificial sequence
<400> 3
   agcttcccga attctgcag 19
<210> 4
   <211> 19
   <212> DNA
   <213> Artificial sequence
<400> 4
   tcgactgcag aattcggga 19
<210> 5
   <211> 19
   <212> DNA
   <213> Artificial sequence
<400> 5
   gtggcagaca caggcagtg 19
<210> 6
   <211> 25
   <212> DNA
   <213> Artificial sequence
<400> 6
   ggccggatcc cagactggcc agtcc 25
<210> 7
   <211> 19
   <212> DNA
   <213> Artificial sequence
<400> 7
   cctgagcagc attccaacg 19
<210> 8
   <211> 24
   <212> DNA
   <213> Artificial sequence
<400> 8
   cttcttggtc tcctcagcat tcac 24
<210> 9
   <211> 20
   <212> DNA
   <213> Artificial sequence
<400> 9
   gtgattgtca ccatggccag 20
<210> 10
   <211> 20
   <212> DNA
   <213> Artificial sequence
<400> 10
   gaaggagttc ttcaggaagc 20
<210> 11
   <211> 30
   <212> DNA
   <213> Artificial sequence
<400> 11
   ccgggaattc ctgagcagca ttccaacgta 30
<210> 12
   <211> 28
   <212> DNA
   <213> Artificial sequence
<400> 12
   ccggggatcc agcccttcaa gttccggc 28
<210> 13
   <211> 33
   <212> DNA
   <213> Artificial sequence
<400> 13
   ccggggatcc agatttctta tttttgtaga tgc 33
<210> 14
   <211> 29
   <212> DNA
   <213> Artificial sequence
<400> 14
   ccgggaattc tcccctgagg agattgagg 29
<210> 15
   <211> 35
   <212> DNA
   <213> Artificial sequence
<400> 15
   ccgggaattc tacaaaaata agaaatctca tgaac 35
<210> 16
   <211> 28
   <212> DNA
   <213> Artificial sequence
<400> 16
   ccggggatcc ggaccagctg atcccctg 28
<210> 17
   <211> 20
   <212> DNA
   <213> Artificial sequence
<400> 17
   gtgggctgag gcacagactg 20
<210> 18
   <211> 24
   <212> DNA
   <213> Artificial sequence
<400> 18
   ccaatcatga agataatgaa aggc 24
<210> 19
   <211> 10
   <212> DNA
   <213> Artificial sequence
<400> 19
   aattcccggg 10
<210> 20
   <211> 9
   <212> DNA
   <213> Artificial sequence
<400> 20
   gatcccggg 9
<210> 21
   <211> 12
   <212> DNA
   <213> Artificial sequence
<400> 21
   tatggaattc gc 12
<210> 22
   <211> 14
   <212> DNA
   <213> Artificial sequence
<400> 22
   catggcgaat tcca 14
<210> 23
   <211> 28
   <212> DNA
   <213> Artificial sequence
<400> 23
   ccgggaattc ggaccagctg atcccctg 28
<210> 24
   <211> 30
   <212> DNA
   <213> Artificial sequence
<400> 24
   ccgggaattc atggccagca agaggaaatc 30
<210> 25
   <211> 29
   <212> DNA
   <213> Artificial sequence
<400> 25
   ccggggatcc cagggggatc atcactttg 29
<210> 26
   <211> 29
   <212> DNA
   <213> Artificial sequence
<400> 26
   ccggggatcc tggcttggcc acgttccac 29
<210> 27
   <211> 29
   <212> DNA
   <213> Artificial sequence
<400> 27
   ccggggatcc tggcttggcc acgttccac 29
<210> 28
   <211> 32
   <212> DNA
   <213> Artificial sequence
<400> 28
   ccggggatcc tgggtcttta ttaaagtctg tg 32
<210> 29
   <211> 30
   <212> DNA
   <213> Artificial sequence
<400> 29
   ccgggaattc acctttgtat gcagtgggtg 30
<210> 30
   <211> 30
   <212> DNA
   <213> Artificial sequence
<400> 30
   ccgggaattc acctttgtat gcagtgggtg 30
<210> 31
   <211> 30
   <212> DNA
   <213> Artificial sequence
<400> 31
   ccgggaattc acctttgtat gcagtgggtg 30
<210> 32
   <211> 30
   <212> DNA
   <213> Artificial sequence
<400> 32
   ccgggaattc acctttgtat gcagtgggtg 30
<210> 33
   <211> 27
   <212> DNA
   <213> Artificial sequence
<400> 33
   aattccacca cactggatcc ctggtac 27
<210> 34
   <211> 25
   <212> DNA
   <213> Artificial sequence
<400> 34
   ggcatcttgc aacaccacag tcttc 25
<210> 35
   <211> 25
   <212> DNA
   <213> Artificial sequence
<400> 35
   catgcatggt gtggtggatt tcctc 25